(19) Europäisches Patentamt
European Patent Office
Office européen des brevets

(11) Publication number: **0 106 140**
**B1**

(12) **EUROPEAN PATENT SPECIFICATION**

(45) Date of publication of patent specification: **17.08.88**

(21) Application number: **83108935.4**

(22) Date of filing: **09.09.83**

(51) Int. Cl.⁴: **C 07 C 69/76**, C 07 D 213/30, C 07 D 257/06, A 61 K 31/44, A 61 K 31/235, A 61 K 31/41 // C07C103/22, C07C121/64

(54) **Benzoic acid derivatives.**

(30) Priority: **10.09.82 US 416482**

(43) Date of publication of application: **25.04.84 Bulletin 84/17**

(45) Publication of the grant of the patent: **17.08.88 Bulletin 88/33**

(84) Designated Contracting States: **BE CH DE FR GB IT LI NL SE**

(56) References cited: **EP-A-0 059 983**

(73) Proprietor: **THE WELLCOME FOUNDATION LIMITED**
**183-193 Euston Road**
**London NW1 2BP (GB)**

(72) Inventor: **Bell, Lawrence Nathaniel**
**4006 Spruce Drive**
**Raleigh, NC 27612 (US)**
Inventor: **Hodgson, Gordon Lewis, Jr.**
**115 Radcliffe Circle**
**Durham, NC 27713 (US)**
Inventor: **Burke, Michael Thomas**
**3803 Hillgrand Drive**
**Durham, NC 27705 (US)**
Inventor: **Shumaker, Thomas Kevin**
**1601 James Street**
**Durham, NC 27707 (US)**

(74) Representative: **Sandmair, Kurt, Dr. et al**
**Patentanwälte Schwabe, Sandmair, Marx**
**Stuntzstrasse 16**
**D-8000 München 80 (DE)**

**EP 0 106 140 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to derivatives of benzoic acid, to pharmaceutical formulations containing them, to processes for their preparation and to the use thereof in human and veterinary medicine. More specifically the invention relates to 2-(substituted phenyl)-benzoic acid derivatives and their use in the control or lowering of blood lipids.

Atherosclerosis is a pathological condition which is a major cause of the occlusive vascular diseases resulting in myocardial infarction and stroke. This condition is associated with a deposition of blood lipids in the wall of major arteries and is known to occur more frequently in subjects whose blood lipids levels are elevated from established norms. The distribution of lipids in the various lipoprotein components of blood determines the degree of risk/benefit of these materials. High levels of cholesterol in the "low" and "very-low" density lipoprotein are associated with enhanced risk of atherosclerotic coronary artery disease while high levels of the "high density" lipoprotein-cholesterol are associated with reduced risks of this disease. Elevation in blood triglyceride levels has also been implicated as a factor which increases the risk of developing these vascular diseases. The strong association between the elevation of specific components of blood lipids and these major cardiovascular diseases has led to attempts to control blood lipid levels by management of diet and through drug intervention. It has been found that certain compounds, those of formula (I) below, are effective in reducing the amount of these lipoprotein components in blood which have been associated with atherosclerosis.

The invention accordingly provides, in a first aspect, compounds of formula (I) below, together with pharmaceutically acceptable salts thereof for use in human and veterinary medicine.

Compounds of formula (I) are:—

$$Ar^1—Ar^2 \qquad (I)$$

Wherein

$Ar^1$ is selected from

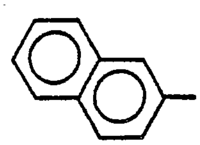 ; 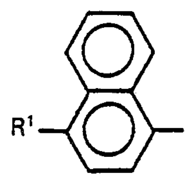 or 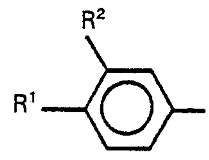

$Ar^2$ is selected from

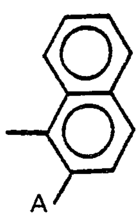 ; 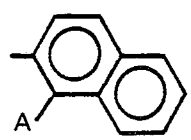 ; 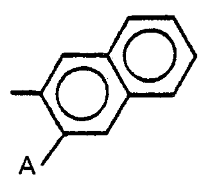 or 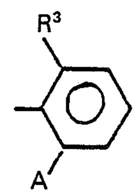

$R^1$ is selected from $C_{1-5}$ alkyl, halogen, perhalo-$C_{1-4}$ alkyl (such as trihalomethyl), $C_{1-4}$ alkoxy, phenyl, $C_{1-4}$ acyl, $C_{1-6}$ alkoxy carbonyl, amino, hydroxy, $SO_2NR^4R^5$, $SCF_3$ or $SOR^6$;

$R^2$ is hydrogen or $C_{1-4}$ alkyl;

$R^3$ is hydrogen or $C_{1-4}$ alkoxy;

$R^4$ and $R^5$ are the same or different and can be $C_{1-4}$ alkyl;

$R^6$ is straight or branched $C_{1-6}$ alkyl;

A is $—CO_2Alk$, 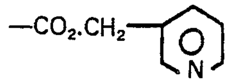 or 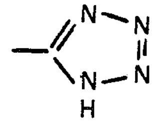 ; and Alk is $C_{1-6}$alkyl

and pharmaceutically acceptable salts thereof.

Preferred compounds within the scope of formula (I) are those of formula (IA) or (IB):

(IA) 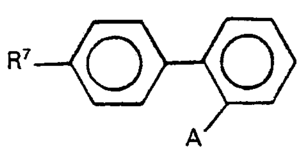 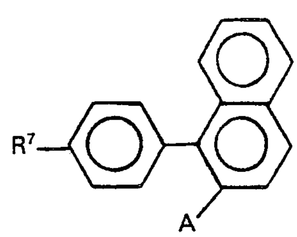 (IB)

wherein A is as defined in formula (I) above and $R^7$ is $C_{1-5}$ alkyl, halogen, perhalo-$C_{1-4}$ alkyl, (such as trihalomethyl), $C_{1-4}$ alkoxy, phenyl, $C_{1-4}$ acyl, $C_{1-6}$ alkoxy carbonyl, amino or hydroxy.

Preferably, $R^7$ is selected from $C_{1-5}$ alkyl, halogen, $C_{1-4}$ alkoxy, phenyl or trihalomethyl. In particular, compounds in which $R^7$ is selected from $C_{2-5}$ alkyl or trihalomethyl are considered advantageous.

When A is $CO_2Alk$, Alk is preferably $C_{1-4}$ alkyl, for example methyl or ethyl.

Compounds wherein A is

$CO_2Et$, $CO_2CH_2$ 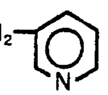 or 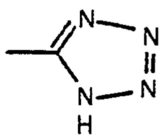

form one particular sub-class of compounds of formula (I). Specific compounds within the scope of formula (I) include for example:—

Methyl 2-(4-trifluoromethylphenyl)benzoate;
Ethyl 2-(4-trifluoromethylphenyl)benzoate;
3-Pyridylmethyl 2-(4-trifluoromethylphenyl)benzoate; and
5-(4′-Trifluoromethylphenyl-2-biphenyl)-tetrazole.

Suitable pharmaceutically acceptable acid addition salts include, for example, those derived from the alkali metals (such as sodium or potassium) or alkaline earth metals (such as calcium or magnesium) and ammonium salts (such as $NR_4^+$ wherein R is hydrogen or $C_{1-4}$ alkyl, eg $NH^+{}_4$).

The compounds of formula (I) are useful in the treatment or prophylaxis of any condition in which the underlying aetiology is associated with elevated blood lipid levels. Thus the compounds of formula (I) are useful in the treatment or prophylaxis of atherosclerosis, reclusive vascular diseases and for the reduction or control of levels of blood lipids such as the triglycerols and cholesterol.

Published European Patent Application EP—A—59983 discloses the compounds of formula (I) wherein A is an ester group. There was no teaching, however, that the compounds exhibited pharmacological activity.

The amount of active compound required for use in the above conditions will vary both with the route of administration, the condition under treatment and the mammal undergoing treatment, and is ultimately at the discretion of the physician. A suitable oral dose of the active compound for a mammal is in the range of from 1 to 40 mg per kilogram body weight per day; preferably in the range of 2 to 10 mg/kg bodyweight, a typical dose for a human recipient being about 4 mg/kg bodyweight per day.

The desired dose is preferably presented as from one to three sub-doses administered at appropriate intervals throughout the day. Where two sub-doses are employed, each will preferably lie in the range of from 1 to 100 mg/kg bodyweight; a typical sub-dose for a human recipient being about 2 mg/kg bodyweight.

While it is possible that the compounds of formula (I) may be administered as the raw chemical it is preferable to present the active ingredient as a pharmaceutical or veterinary formulation.

Pharmaceutical formulations comprise the active compounds together with one or more pharmaceutically acceptable carriers therefor and optionally other therapeutic and/or prophylactic ingredients. The carrier(s) must be 'acceptable' in the sense of being compatible with the other ingredients of the formulation and not deleterious to the recipient thereof.

The invention thus provides in a further aspect a pharmaceutical formulation comprising a compound of formula (I) or a pharmaceutically acceptable salt thereof together with a pharmaceutically acceptable carrier therefor.

The active compound(s) may conveniently be presented (as a pharmaceutical formulation) in unit dosage form. A convenient unit dose formulation contains the active ingredient compound(s) in an amount of from 1 mg to 10 mg.

Pharmaceutical formulations include those suitable for oral, rectal or parenteral (including intramuscular and intravenous) administration, although oral is the preferred route. The formulations may, where appropriate, be conveniently presented in discrete dosage units and may be prepared by any of the methods well known in the art of pharmacy. All methods include the step of bringing into association the active compound with liquid carriers or finely divided solid carriers of both and then, if necessary, shaping the product into the desired formulation.

There is thus provided in a further aspect a method for the preparation of a pharmaceutical formulation comprising a compound of formula I (or a pharmaceutically acceptable salt thereof) together with a pharmaceutically acceptable carrier therefor, which method comprises bringing into association the active compound of formula (I) and the carrier therefor.

Pharmaceutical formulations suitable for oral administration wherein the carrier is a solid are most preferably presented as unit dose formulations such as boluses, capsules, cachets or tablets each containing a predetermined amount of the active ingredient. A tablet may be made by compression or moulding, optionally with one or more accessory ingredients. Compressed tablets may be prepared by compressing in a suitable machine the active compound in a free-flowing form such as a powder or granules optionally mixed with a binder, lubricant, inert diluent, lubricating, surface active or dispersing

agent. Moulded tablets may be made by moulding an inert liquid diluent. Tablets may be optionally coated and, if uncoated, may optionally scored. Capsules may be prepared by filling the active compound, either alone or in admixture with one or more accessory ingredients, into the capsule cases and then sealing then in the usual manner. Cachets are analogous to capsules wherein the active ingredient together with any accessory ingredient(s) is sealed in a rice paper envelope.

Pharmaceutical formulations suitable for oral administration wherein the carrier is a liquid may be presented as a solution or a suspension in an aqueous liquid or a non-aqueous liquid, or as an oil-in-water or water-in-oil liquid emulsion.

Pharmaceutical formulations suitable for rectal administration wherein the carrier is a solid are most preferably presented as unit dose suppositories. Suitable carriers include cocoa butter and other materials commonly used in the art, and the suppositories may be conveniently formed by admixture of the active compound with the softened or melted carrier(s) followed by chilling and shaping moulds.

Pharmaceutical formulations suitable for parenteral administration include sterile solutions or suspensions of the active compound in aqueous or oleaginous vehicles. Such preparations are conveniently presented in unit dose or multi-dose containers which are sealed after introduction of the formulation until required for use.

It should be understood that in addition to the aforementioned carrier ingredients the pharmaceutical formulations described above may include, as appropriate, one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

Compounds of formula (I) and their salts may be prepared by any method well known in the art for preparation of compounds of analogous structure.

For example, compounds of formula (I) may be prepared by derivatisation of the corresponding carboxylic acid. The carboxylic acids themselves may be obtained by methods known in the art, for example European Patent Application Publication No 0059983 (which is incorporated herein by reference).

Compounds of formula (I) wherein A is a tetrazole residue may also be prepared by reaction of the corresponding nitrile with an appropriate reagent, for example azide.

The following Examples illustrate the invention:—

## Example 1

Preparation of Ethyl 2-(4-trifluoromethylphenyl)benzoate

A solution of 2-(4-trifluoromethylphenyl)benzoic acid (3.0 g, 0.0113 mole) and 15 drops concentrated sulfuric acid in 250 ml absolute ethanol was heated to reflux in a Soxhlet extractor filled with dry 3A° Molecular Sieve (Mallinckrodt) for 7 days. The pH of the reaction mixture was brought to ≃7 with the addition of 1N NaOH and the mixture was evaporated to a residue. The residue was dissolved in ether and filtered. The filtrate was evaporated to an oil. The oil was chromatographed using petroleum ether:ethyl acetate to afford ethyl-2-(4-trifluoromethylphenyl)benzoate as an oil (yield 75%).

Elemental Analysis: Calculated for $C_{16}H_{13}F_3O_2$: C, 65.30; H, 4.45.
Found: C, 65,10; H, 4.55.

TLC (lined tank), 1:1:methylene chloride:petroleum ether, 1 spot, $R_f$ 0.55, Silica Gel 60 (Trade Name) plate.

## Example 2

By the method of Example 1, methyl 2-(4-trifluoromethylphenyl)benzoate, was also prepared, as an oil.

## Example 3

Preparation of 3-Pyridylmethyl-2-(4-trifluoromethylphenyl)benzoate

A solution of 2-(4-trifluoromethylphenyl)benzoic acid (2.6 g 0.01 mole) in 100 mL thionyl chloride was stirred at room temperature for 16 hr. The reaction mixture was evaporated under vacuum to an oil. The oil was dissolved in 100 mL toluene to which was added 3-pyridylcarbinol (1.6 g, 0.015 mole) and triethylamine (1 mL).

The reaction mixture was heated and stirred under nitrogen for 16 hr. The solution was then cooled, filtered and evaporated under reduced pressure to an oil. The oil was chromatographed in 20% ethyl acetate:petroleum ether to afford 3-pyridylmethyl-2-(4-trifluoromethylphenyl)-benzoate as an oil (yield 86%).

Elemental Analysis: Calculated for $C_{20}H_{14}NO_2F_3$: C, 67.22; H, 3.95; N, 3.92.
Found: C, 66.96; H, 4.00; N, 3.85.

TLC (lined tank), 1:1: ethyl acetate:petroleum ether, 1 spot, $R_f$ 0.55, Silica Gel 60 (Trade Name) plate.

## Example 4

5-(4'-Trifluoromethyl-2-biphenyl)tetrazole

*(a) Preparation of 2-(4-Trifluoromethylphenyl)benzamide*

A solution of 2-(4-Trifluoromethylphenyl)benzoic acid (3.0 g, 0.011 mole) in 100 mL thionyl chloride was stirred at room temperature for 2 hr. The reaction mixture was evaporated, under reduced pressure,

with heating, to a residue. The residue was dissolved in 250 mL aqueous ammonium hydroxide and stirred for 16 hr. The reaction mixture was filtered to afford 2-(4-trifluoromethylphenyl)benzamide as a white solid (yield 90%, m.p. 134—137°).

Elemental Analysis: Calculated for $C_{14}H_{10}NOF_3$: C, 63.40; H, 3.80; N, 5.28.
Found: C, 63.09; H, 3.70; N, 5.31.

TLC (lined tank), 10% methanol/chloroform, 1 spot, $R_f$ = 0.65, Silica Gel 60 (Trade Name).

*(b) Preparation of 2-(4-Trifluoromethylphenyl)benzonitrile*

A solution of thionyl chloride (6 g, 0.05 mole) and dimethylformamide (3.6 g, 0.05 mole) was stirred at 0° for 20 minutes. 2-(4-Trifluoromethylphenyl)benzamide (1.5 g, 0.0057 mole) was then added and the reaction stirred at 0° for 2 hr. The reaction was then allowed to warm to room temperature and stirring was continued overnight. The reaction was then poured over ice and filtered to afford 2-(4-trifluoromethylphenyl)benzonitrile as a solid (yield 100%).

Infrared Analysis: sharp peak at 2210 $cm^{-1}$.

*(c) Preparation of 5-(4'-Trifluoromethyl-2-biphenyl)tetrazole*

A mixture of 2-(4-trifluoromethylphenyl)benzonitrile (3.0 g, 0.012 mole), lithium chloride (1.0 g, 0.024 mole), sodium azide (1.74 g, 0.0266 mole) and aniline hydrochloride (0.32 g, 0.0024 mole) in 15 mL dimethylformamide was heated to 100° for 12 days. The reaction was then evaporated, under reduced pressure, to a residue. The residue was dissolved in 100 mL water and extracted twice with 100 mL methylene chloride. The organic extracts were combined and washed once with 100 mL brine, then dried with sodium sulfate and evaporated, under reduced pressure, to an oil. The oil was chromatographed in 1:1:ethyl acetate:petroleum ether to yield a solid which was recrystallized in ether/petroleum ether to afford 5-(4-trifluoromethyl-2-biphenyl)tetrazole (yield 51%, m.p. 125—127°).

Elemental Analysis: Calculated for $C_{14}H_9F_3N_4$: C, 57.93; H, 3.12; N, 19.30.
Found: C, 57.83; H, 3.15; N, 19.32.

TLC (lined tank), 10% methanol/chloroform, 1 spot, $R_f$ = 0.29, Silica Gel 60 (Trade Name) plate.

## Example 5

Effect of Compounds of formula (IA) on Cholesterol and Triglyceride levels in rats

Various compounds of formula (IA) were assessed for their ability to reduce cholesterol and triglyceride levels in rats as follows:—

Hypercholesterolemia was produced in male rats after 3 days in a diet of 0.4% cholesterol and 0.2% sodium cholate. Each group contained 4 rats. Compounds tested were given orally twice daily for 3 days and once on day 4. Control animals received the vehicle, methyl cellulose. The cholesterol-containing diet and compound treatment were started at the same time. Animals were bled before dietary and compound treatment and 3 hr after the last dose of compound on day 4. All bleedings were after a 4 hr fast. Total serum cholesterol, triglyceride and high density lipoprotein (HDL) cholesterol were determined by the procedure and test kit supplied by Dow Diagnostics (Trademark of the Dow Chemical Co. Laboratories, Indianapolis, IN). Very low and low density lipoprotein (VLDL & LDL) cholesterol were determined by the difference between total and HDL cholesterol.

The compounds were compared in efficacy to clofibrate. The results are shown in Table I.

TABLE (I)

| Compound of formula (IA) R | A | Cholesterol lowering | Triglyceride lowering |
|---|---|---|---|
| $CF_3$ | $CO_2Me$ | | |
| $CF_3$ | $CO_2Et$ | 35% at 25 mg/kg bid | 39% at 25 mg/kg bid |

Example 6
Pharmaceutical Formulation

| | | |
|---|---|---|
| a. | Capsule | |
| | Compound of formula (I) | 150.0 mg |
| | Corn Starch | 45.0 mg |
| | Stearic Acid | 12.0 mg |
| | Lactose | 93.0 mg |
| b. | Syrup | |
| | Compound of formula (I) | 162.4 mg |
| | Glycerin | 500.0 mg |
| | Sucrose | 3,500.0 mg |
| | Flavouring Agent | q.s. |
| | Colouring Agent | q.s. |
| | Preserving Agent | 0.1% |
| | Purified Water | q.s. to 5.0 ml |
| c. | Tablet | |
| | Compound of formula (I) | 162.4 mg |
| | Corn Starch | 30.0 mg |
| | Lactose | 87.6 mg |
| | Magnesium Stearate | 3.0 mg |
| | Polyvinylpyrrolidone | 6.0 mg |
| | Stearic Acid | 12.0 mg |
| d. | Tablet | |
| | Compound of formula (I) | 150.0 mg |
| | Corn Starch | 30.0 mg |
| | Lactose | 100.0 mg |
| | Magnesium Stearate | 2.0 mg |
| | Polyvinylpyrrolidone | 6.0 mg |
| | Stearic Acid | 12.0 mg |

**Claims**

1. A compound of the formula (I)

$$Ar^1—Ar^2 \quad (I)$$

wherein:—

$Ar^1$ is selected from

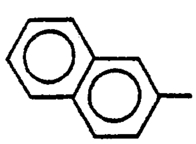 ; 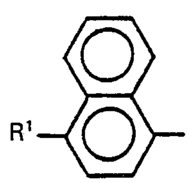 or 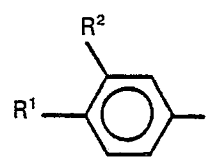

$Ar^2$ is selected from

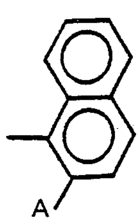 ; 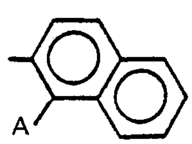 ; 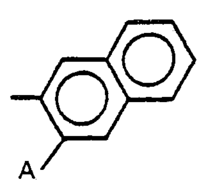 or 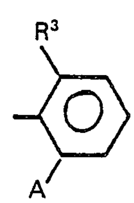

and

$R^1$ is selected from $C_{1-5}$ alkyl, halogen, perhalo-$C_{1-4}$ alkyl (such as trihalomethyl), $C_{1-4}$ alkoxy, phenyl, $C_{1-4}$ acyl, $C_{1-6}$ alkoxy carbonyl, amino, hydroxy, $SO_2NR^4R^5$, $SCF_3$ or $SOR^6$;

$R^2$ is hydrogen or $C_{1-4}$ alkyl;

$R^3$ is hydrogen or $C_{1-4}$ alkoxy;

$R^4$ and $R^5$ are the same or different and can be $C_{1-4}$ alkyl;

$R^6$ is straight or branched $C_{1-6}$ alkyl;

A is

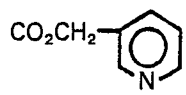 or 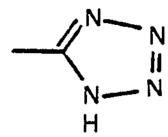

and pharmaceutically acceptable salts thereof.

2. A compound of formula (I) as claimed in claim 1 which compound is a compound of formula (IA),

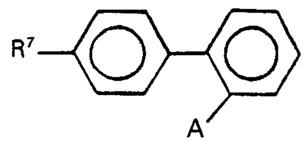

(IA)

wherein A is as defined in claim 1 and $R^7$ is $C_{1-5}$ alkyl, halogen, perhalo-$C_{1-4}$ alkyl, $C_{1-4}$ alkoxy, phenyl, $C_{1-4}$ acyl, $C_{1-6}$ alkoxycarbonyl, amino or hydroxy.

3. A compound of formula (I) as claimed in claim 1 which compound is a compound of formula (IB),

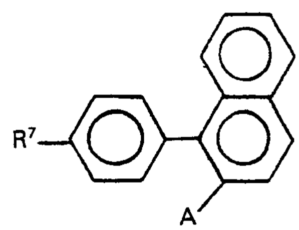

(IB)

wherein A is as defined in claim 1 and $R^7$ is $C_{1-5}$ alkyl, halogen, perhalo-$C_{1-4}$ alkyl, $C_{1-4}$ alkoxy, phenyl, $C_{1-4}$ acyl, $C_{1-6}$ alkoxy carbonyl, amino or hydroxy.

4. A compound is claimed in claim 2 or claim 3 wherein $R^7$ is selected from $C_{1-5}$ alkyl, halogen, $C_{1-4}$ alkoxy, phenyl or trihalomethyl.

5. A compound as claimed in any one of claims 1 to 4 wherein $R^1$ is $C_{2-5}$ alkyl or trihalomethyl.

6. A compound of formula (I) selected from, 3-pyridylmethyl 2-(4-trifluoromethylphenyl)benzoate, 5-(4'-trifluoromethyl-2-biphenyl)-tetrazole.

7. A pharmaceutical formulation comprising a compound of formula (I) as defined in claims 1 to 6 including a compound of formula I wherein A represents $CO_2Alk$, wherein Alk is $C_{1-6}$ alkyl.

8. A pharmaceutical formulation according to claim 7 wherein the compound is a $C_{1-6}$ alkylester of 2-(4-trifluoromethylphenyl)benzoic acid.

9. A pharmaceutical formulation according to claim 7 wherein the compound is selected from methyl 2-(4-trifluoromethylphenyl)benzoate and ethyl 2-(4-trifluoromethylphenyl)benzoate.

10. A compound of formula I as defined in any one of claims 1 to 6 including a compound of formula (I) wherein A represents $CO_2Alk$ and Alk is $C_{1-6}$ alkyl for use in therapy.

11. The compound according to claim 10 which is a $C_{1-6}$ alkyl ester of 2-(4-trifluoromethylphenyl)-benzoic acid, for use in therapy.

12. The compound according to claim 10 or 11 which is methyl 2-(4-trifluoromethylphenyl)benzoate or ethyl 2-(4-trifluoromethylphenyl)benzoate, for use in therapy.

**Patentansprüche**

1. Verbindung der Formel (I)

$$Ar^1—Ar^2 \qquad (I)$$

worin

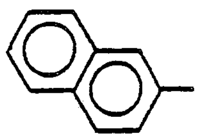 ; 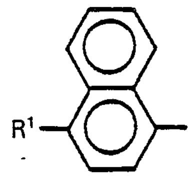 oder 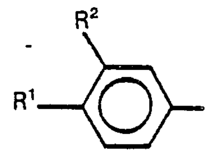

$Ar^2$ ausgewählt ist aus

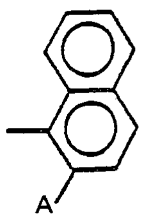 ; 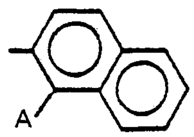 ; 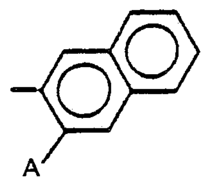 oder 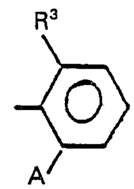

und

$R^1$ ausgewählt ist aus $C_{1-5}$ Alkyl, Halogen, Perhalo-$C_{1-4}$ Alkyl, (wie Trihalomethyl), $C_{1-4}$ Alkoxy, Phenyl, $C_{1-4}$ Acyl, $C_{1-6}$ Alkoxycarbonyl, Amino, Hydroxy, $SO_2NR^4R^5$, $SCF_3$ oder $SOR^6$;

$R^2$ Wasserstoff oder $C_{1-4}$ Alkyl bedeutet;

$R^3$ Wasserstoff oder $C_{1-4}$ Alkoxy bedeutet;

$R^4$ und $R^5$, die gleich oder verschieden sind, $C_{1-4}$ Alkyl bedeuten;

$R^6$ ein geradkettiges oder verzweigtes $C_{1-6}$ Alkyl bedeutet;

A

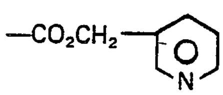 oder 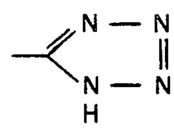

bedeutet;

und pharmazeutisch annehmbare Salze davon.

2. Verbindung der Formel (I) nach Anspruch 1, wobei die Verbindung eine Verbindung der Formel (IA) ist

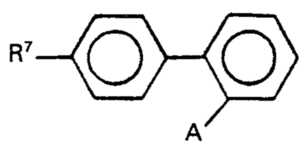

(IA)

worin A die in Anspruch 1 gegebene Bedeutung aufweist und $R^7$ $C_{1-5}$ Alkyl, Halogen, Perhalo-$C_{1-4}$ Alkyl, $C_{1-4}$ Alkoxy, Phenyl, $C_{1-4}$ Acyl, $C_{1-6}$ Alkoxycarbonyl, Amino oder Hydroxy bedeutet.

3. Verbindung der Formel (I) nach Anspruch 1, wobei die Verbindung eine Verbindung der Formel (IB) ist

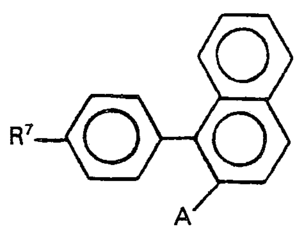

(IB)

worin A die in Anspruch 1 gegebene Bedeutung aufweist und $R^7$ $C_{1-5}$ Alkyl, Halogen, Perhalo-$C_{1-4}$ Alkyl, $C_{1-4}$ Alkoxy, Phenyl, $C_{1-4}$ Acyl, $C_{1-6}$ Alkoxycarbonyl, Amino oder Hydroxy bedeutet.

4. Verbindung nach Anspruch 2 oder 3, worin $R^7$ aus $C_{1-5}$ Alkyl, Halogen, $C_{1-4}$ Alkoxy, Phenyl oder Trihalomethyl ausgewählt ist.

5. Verbindung nach einem der Ansprüche 1 bis 4, worin $R^1$ $C_{2-5}$ Alkyl oder Trihalomethyl ist.

6. Verbindung der Formel (I) ausgewählt aus 3-Pyridylmethyl-2-(4-trifluoromethylphenyl)benzoat, 5-(4'-Trifluoromethyl-2-biphenyl)-tetrazol.

7. Pharmazeutische Formulierung enthaltend eine Verbindung der Formel (I) nach Anspruch 1 bis 6, umfassend eine Verbindung der Formel (I), worin A $CO_2Alk$ bedeutet, worin Alk $C_{1-6}$ Alkyl ist.

8. Pharmazeutische Formulierung nach Anspruch 7, worin die Verbindung ein $C_{1-6}$ Alkylester von 2-(4-Trifluoromethylphenyl)benzoesäure ist.

9. Pharmazeutische Formulierung nach Anspruch 7, worin die Verbindung aus Methyl-2-(4-Trifluoromethylphenyl)benzoat und Ethyl-2-(4-Trifluoromethylphenyl)benzoat ausgewählt ist.

10. Verbindung der Formel (I) nach einem der Ansprüche 1 bis 6, umfassend eine Verbindung der Formel (I), worin A $CO_2Alk$ bedeutet und Alk $C_{1-6}$ Alkyl ist, zur Verwendung in der Therapie.

11. Verbindung nach Anspruch 10, worin die Verbindung ein $C_{1-6}$ Alkylester von 2-(4-Trifluoromethylphenyl)benzoesäure ist zur Verwendung in der Therapie.

12. Verbindung nach Anspruch 10 oder 11, worin die Verbindung Methyl-2-(4-Trifluoromethylphenyl)-benzoat oder Ethyl-2-(4-trifluoromethylphenyl)benzoat ist, zur Verwendung in der Therapie.

**Revendications**

1. Composé de formule (I)

$$Ar^1—Ar^2 \qquad (I)$$

où

$Ar^1$ est choisi parmi

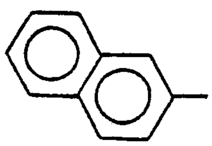 ; 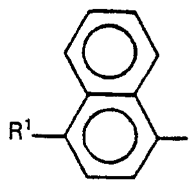 et 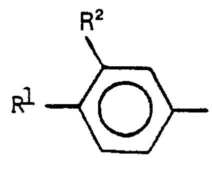

$Ar^2$ est choisi parmi

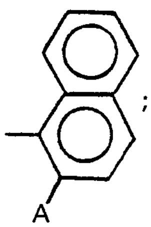 ; 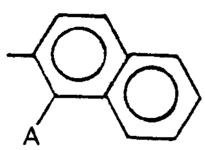 ; 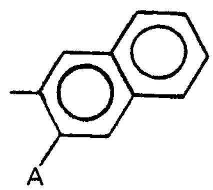 et 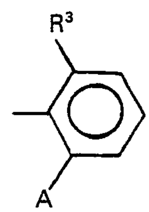

$R^1$ est choisi parmi $C_{1-5}$ alcoyle, halogène, perhalo-$C_{1-4}$ alcoyle (comme trihalométhyle), $C_{1-4}$ alcoxy, phényle, $C_{1-4}$ acyle, $C_{1-6}$ alcoxycarbonyle, amino, hydroxyle, $SO_2NR^4R^5$, $SCF_3$ et $SOR^6$;

$R^2$ est hydrogène ou $C_{1-4}$ alcoyle;

$R^3$ est hydrogène ou $C_{1-4}$ alcoxy;

$R^4$ et $R^5$ sont identiques ou différents et peuvent être $C_{1-4}$ alcoyle;

$R^6$ est $C_{1-6}$ alcoyle en chaîne droite ou ramifiée;

A est

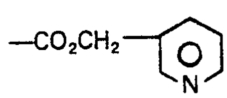 ou 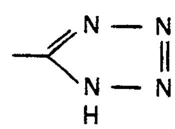

et ses sels pharmaceutiquement acceptables.

2. Composé de formule (I) suivant la revendication 1, lequel est un composé de formule (IA)

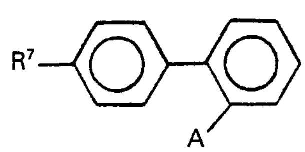 (IA)

où A est tel que défini dans la revendication 1 et $R^7$ est $C_{1-5}$ alcoyle, halogène, perhalo-$C_{1-4}$ alcoyle, $C_{1-4}$ alcoxy, phényle, $C_{1-4}$ acyle, $C_{1-6}$ alcoxycarbonyle, amino ou hydroxyle.

3. Composé de formule (I) suivant la revendication 1, lequel est un composé de formule (IB)

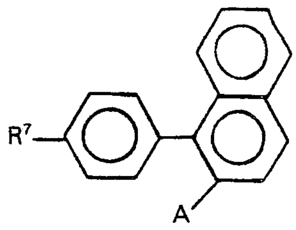

(IB)

où A est tel que défini dans la revendication 1 et $R^7$ est $C_{1-5}$ alcoyle, halogène, perhalo-$C_{1-4}$ alcoyle, $C_{1-4}$ alcoxy, phényle, $C_{1-4}$ acyle, $C_{1-6}$ alcoxycarbonyle, amino ou hydroxyle.

4. Composé suivant la revendication 2 ou 3, dans lequel $R^7$ est choisi parmi $C_{1-5}$ alcoyle, halogène, $C_{1-4}$ alcoxy, phényle et trihalométhyle.

5. Composé suivant l'une quelconque des revendications 1 à 4, dans lequel $R^1$ est $C_{2-5}$ alcoyle ou trihalométhyle.

6. Composé de formule (I) choisi entre le 2-(4-trifluorométhylphényl)benzoate de 3-pyridylméthyle et le 5-(4'-trifluorométhyl-2-biphényl)tétrazole.

7. Composition pharmaceutique comprenant un composé de formule (I) tel que défini dans les revendications 1 à 6, qui comprend un composé de formule I où A représente $CO_2Alk$, où Alk est $C_{1-6}$ alcoyle.

8. Composition pharmaceutique suivant la revendication 7, dans laquelle le composé est un ester $C_{1-6}$ alcoylique de l'acide 2-(4-trifluorométhylphényl)benzoïque.

9. Composition pharmaceutique suivant la revendication 7, dans laquelle le composé est choisi entre le 2-(4-trifluorométhylphényl)benzoate de méthyle et le 2-(4-trifluorométhylphényl)benzoate d'éthyle.

10. Composé de formule I tel que défini dans l'une quelconque des revendications 1 à 6, qui est un composé de formule (I) où A représente $CO_2Alk$ et Alk est $C_{1-6}$ alcoyle, pour l'utilisation en thérapeutique.

11. Composé suivant la revendication 10, qui est un ester $C_{1-6}$ alcoylique de l'acide 2-(4-trifluorométhylphényl)benzoïque, pour l'utilisation en thérapeutique.

12. Composé suivant la revendication 10 ou 11 qui est le 2-(4-trifluorométhylphényl)benzoate de méthyle ou le 2-(4-trifluorométhylphényl)benzoate d'éthyle, pour l'utilisation en thérapeutique.